# EUROPEAN PATENT SPECIFICATION

(11) **EP 1 825 821 B1**
(45) Date of publication and mention of the grant of the patent: **22.09.2010**
(21) Application number: 07004025.8
(22) Date of filing: 27.02.2007
(51) Int. Cl.: A61B 17/115

(54) **Annular disk for reduction of anastomotic tension**
Ringförmige Scheibe zum Reduzieren der anastomotischen Spannung
Disque annulaire pour la réduction de la tension anastomotique

(30) Priority: 28.02.2006 US 365637
(43) Date of publication of application: 29.08.2007
(73) Proprietor: Tyco Healthcare Group LP, Norwalk, CT 06856 (US)
(72) Inventor: Bettuchi, Michael J., Middleton, CT 06457 (US)
(74) Representative: Alcock, David

(56) References cited:
- EP-A- 1 647 231
- EP-A- 1 702 570
- WO-A-03/105698
- WO-A-2006/044160
- WO-A-2006/044490
- DE-A1- 19 924 311
- US-A- 5 915 616

## Description

### BACKGROUND

### Technical Field

The present disclosure relates to annular surgical structures and, more particularly, to adhesive structures, gaskets, disks and the like for use in conjunction with circular stapling devices, for reducing occurrences of anastomotic tension and the like.

### Background of Related Art

Staples have traditionally been used to replace suturing when joining or anastomosing various body structures, such as, for example, the bowel or bronchus. The surgical stapling devices employed to apply these staples are generally designed to simultaneously cut and seal an extended segment of tissue in a patient, thus vastly reducing the time and risks of such procedures.

Linear or annular surgical stapling devices are employed by surgeons to sequentially or simultaneously apply one or more linear rows of surgical fasteners, e.g., staples or two-part fasteners, to body tissue for the purpose of joining segments of body tissue together and/or for the creation of anastomoses. Linear surgical stapling devices generally include a pair of jaws or finger-like structures between which body tissue to be joined is placed. When the surgical stapling device is actuated and/or "fired", firing bars move longitudinally and contact staple drive members in one of the jaws, and surgical staples are pushed through the body tissue and into/against an anvil in the opposite jaw thereby crimping the staples closed. A knife blade may be provided to cut between the rows/lines of staples. Examples of such surgical stapling devices are described in U.S. Patent Nos. 4,354,628, 5,014,899 and 5,040,715.

Annular surgical stapling devices generally include an annular staple cartridge assembly including a plurality of annular rows of staples, typically two, an anvil assembly operatively associated with the annular cartridge assembly, and an annular blade disposed internal of the rows of staples. Examples of such annular surgical stapling devices are described in U.S. Patent Nos. 5,799,857 and 5,915,616 to Robertson et al..

For most procedures, the use of bare staples, with the staples in direct contact with the patient's tissue, is generally acceptable. The integrity of the tissue will normally serve to prevent the staples from tearing out of the tissue and compromising the sealing before healing has occurred. However, in some surgical operations, surgical supports, e.g., meshes, are employed by surgeons to bridge, repair and/or reinforce tissue defects with a patient, especially those occurring in the abdominal wall, chest wall, diaphragm and other musculo-aponeurotic areas of the body. Examples of surgical supports are disclosed in U.S. Patent Nos. 3,054,406, 3,124,136, 4,347,847, 4,655,221, 4,838,884 and 5,002,551.

When the staples are applied in surgical procedures utilizing surgical supports (i.e., reinforcing material), the legs of the staple typically pass from the cartridge jaw through a layer of the surgical support, and through the patient's tissue before encountering the anvil jaw. In an alternative procedure, the legs of the staple typically pass from the cartridge jaw through a first layer of the surgical support, then through the patient's tissue, and finally through a second layer of the surgical support before encountering the anvil jaw. With the staples in place, the stapled tissue is clamped between the layers of the surgical support.

The surgical supports described above are used in conjunction with linear surgical stapling devices. An end-to-end anastomosis stapler such as a Model "EEA^{™}" instrument is available from United States Surgical, a Division of Tyco Health-Care Group, LP, Norwalk, CT and disclosed in U.S. Patent No. 5,392,979 to Green et al.. In general, an end-to-end anastomosis stapler typically places an array of staples into the approximated sections of a patient's bowels or other tubular organs. The resulting anastomosis contains an inverted section of bowel which contains numerous "B" shaped staples to maintain a secure connection between the approximated sections of bowel.

In addition to the use of surgical staples, biological tissue adhesives have been developed for tissue repair and the creation of anastomoses. Generally, biological adhesives bond separated tissues together to aid in the healing process and to enhance the tissue strength. Such adhesives may be used instead of suturing and stapling, for example, in surgical procedures, for the repair of tissue or the creation of anastomoses.

In addition to the use of biological adhesives, following the formation of the anastomosis, a separate instrument or device is used to apply biological sealants to the anastomosis. Typically, in a separate step, the biological sealants are applied to the outer surface of the anastomosis by spraying on, brushing on, swabbing on, any combinations thereof, or any other method contemplated by those skilled in the art. The biological sealants act to reduce and/or stop the incidents of leakage from the anastomosis.

One possible side effect of any end-to-end bowel anastomosis is its tendency to stenos over time, which stenosis can decrease the diameter of the lumen over time. Accordingly, the need exists for a structure which assists in maintaining the lumen of the anastomosed bowel or other tubular organ open over time.

The application of a suitable biocompatible adhesive offers many advantages to the patient and the surgeon alike, such as, for example, the possible reduction in the number of staples used, immediate sealing of the tissue being treated, a strengthening of the anastomosis, and a reduction in the occurrence of bleeding from the blood vessels, leakage through the tissue joint, and stricture. Moreover, use of biocompatible adhesives tends to minimize foreign body reaction and scarring.

An anastomosis is subjected to some degree of tension in the body. The tension places strain on the staples joining the sections of intestinal tissue, especially the radially outward row of staples. As a result, this degree of tension may result in anastomotic leakage occurring between the adjoining sections of intestinal tissue, and/or it may result in a decreased flow of blood to the surgical site thus compromising the rate of healing.

Accordingly, the need exists for annular supports which operate in conjunction with any end-to-end, annular or circular stapling device to reduce the occurrence of anastomoteic tension acting on the surgical staple at the interface between a radially outer surgical staple and the anastomotic tissue.

WO 03/105698 discloses an annular support structure for use with a circular endoscopic stapling instrument having a staple cartridge assembly and an anvil assembly. The support structure comprises a rigid annular ring configured to substantially overlie the at least one annular arrangement of staples of the staple cartridge assembly. The annular ring includes an outer annular wall having a diameter, an inner annular wall spaced a radial distance inward of the outer annular wall and defining a space, an upper wall interconnecting the outer annular wall and the inner annular wall, and a lower wall spaced a distance from the upper wall and interconnecting the outer annular wall and the inner annular wall. The outer annular wall, the inner annular wall and the upper and lower walls define an interior reservoir. The support structure includes a wound closure material retained in the reservoir.

DE 19924311 discloses a clip cutting device to cut body tissue and place a staple on at least one side of a cut line. The device has two moving parts which clamp the body tissue. One part has a clamp head with a number of staples. The other part has a clamp pressure plate to secure the staples to the tissue. At least one of the moving parts has an underlay of collagen and fibrin fleece which extends along the staple arrangement.

### SUMMARY

The present disclosure relates to adhesive disks for use in conjunction with circular stapling devices, for reducing occurrences of anastomotic tension and the like.

According to an aspect of the present disclosure there is provided claim 1.

The present disclosure describes a method of performing an anastomotic procedure on adjacent tissue sections is provided. The method includes the steps of: a) providing a surgical stapling device including an anvil assembly and a body portion, the anvil assembly including an anvil member supported on an anvil shaft and the body portion carrying a plurality of surgical staples arranged in an annular row and a knife; and b) providing an adhesive disk having an outer terminal portion and a substantially centrally located aperture, wherein the outer terminal portion of adhesive disk extends radially outward beyond an outer-most row of the at least one annular row of staples.

The method further includes the steps of: c) inserting the anvil assembly into a first tissue section; d) inserting the body portion into a second tissue section; e) disposing the adhesive disk between the first tissue section and the second tissue section; f) approximating the anvil assembly and body portion with one another so that an end portion of the first tissue section, an end portion of the second tissue section and the adhesive disk are disposed between the anvil member and the body portion, wherein the adhesive disk is disposed between the first tissue section and the second tissue section and the outer terminal edge thereof extends radially outward beyond the outer-most row of the at least one annular row of staples, wherein the adhesive disk adheres the end portions of the first and second tissue sections to one another; g) deploying the staples from the body portion; and h) cutting the first tissue section, the second tissue section, and the adhesive disk with the knife.

The present disclosure also describes a method of joining adjacent tissue sections is provided. The method includes the steps of: a) providing a surgical stapling device including an anvil assembly having an anvil shaft, and a body portion carrying a plurality of surgical staples arranged in an annular row and a knife; and b) providing an adhesive disk having an outer terminal portion which extends radially outward beyond an outer-most row of the at least one annular row of staples.

The described method further includes the steps of: c) inserting the anvil assembly into a first tissue section; d) inserting the body portion into a second tissue section; e) disposing the adhesive disk between the first tissue section and the second tissue section; f) approximating the anvil assembly and body portion with one another so that the first tissue section, the second tissue section and the adhesive disk are disposed between the anvil member and the body portion, wherein the adhesive disk is interposed between the first tissue section and the second tissue section, and wherein the outer terminal portion of the adhesive disk extends radially outward beyond the outer-most row of the at least one annular row of staples; g) deploying the staples from the body portion; and h) cutting the first tissue section, the second tissue section, and the adhesive disk with the knife.

It is envisioned that the adhesive disk may be fabricated from at least one of a bioabsorbable and a non-bioabsorbable material.

The adhesive disk may include a material selected from the group consisting of a sealant, a hemostat, and a medicament.

It is envisioned that at least the portion of the adhesive disk which extends radially outward beyond the outer-most row of the at least one annular row of staples adheres the adjacent tissue sections to one another. The adhesive disk adheres the adjacent tissue sections to one another at least at the location radially outward of the outer-most row of the at least one annular row of staples.

The adhesive disk reduces the tension exhibited on the outer-most row of the at least one annular row of staples when the adjacent tissue sections are pulled away from one another.

### BRIEF DESCRIPTION OF DRAWINGS

The accompanying drawings, which are incorporated in and constitute a part of this specification, illustrate embodiments of the disclosure and, together with a general description of the disclosure given above and the detailed description of the embodiments given below, serve to explain the principles of the disclosure, wherein:

FIG. 1 is a perspective view of an exemplary annular surgical stapling device;

FIG. 2 is a cross-sectional schematic illustration of a pair of adjacent tissue sections joined to one another pursuant to a prior art anastomotic procedure, and exhibiting tension therebetween and on the resulting staple line;

FIG. 3 is a perspective view of an adhesive disk according to an embodiment of the present disclosure;

FIG. 4 is a perspective view of the intestinal area of a patient, illustrating a method of positioning the adhesive disk of FIG. 3 on the anvil rod of the surgical stapling device of FIG. 1;

FIG. 5 is a schematic perspective view of the intestinal area of FIG. 4, illustrating the anvil rod mounted to the annular stapling device and having the adhesive disk of FIG. 3 disposed therebetween; and

FIG. 6 is a cross-sectional schematic illustration of the pair of adjacent tissue sections of the intestinal area of FIGS. 4 and 5, joined to one another pursuant to a method of the present disclosure, and exhibiting a reduction of tension therebetween and on the resulting staple line.

### DETAILED DESCRIPTION OF EMBODIMENTS

Embodiments of the presently disclosed center hub will now be described in detail with reference to the drawing figures wherein like reference numerals identify similar or identical elements. As used herein and as is traditional, the term "distal" refers to that portion which is furthest from the user while the term "proximal" refers to that portion which is closest to the user.

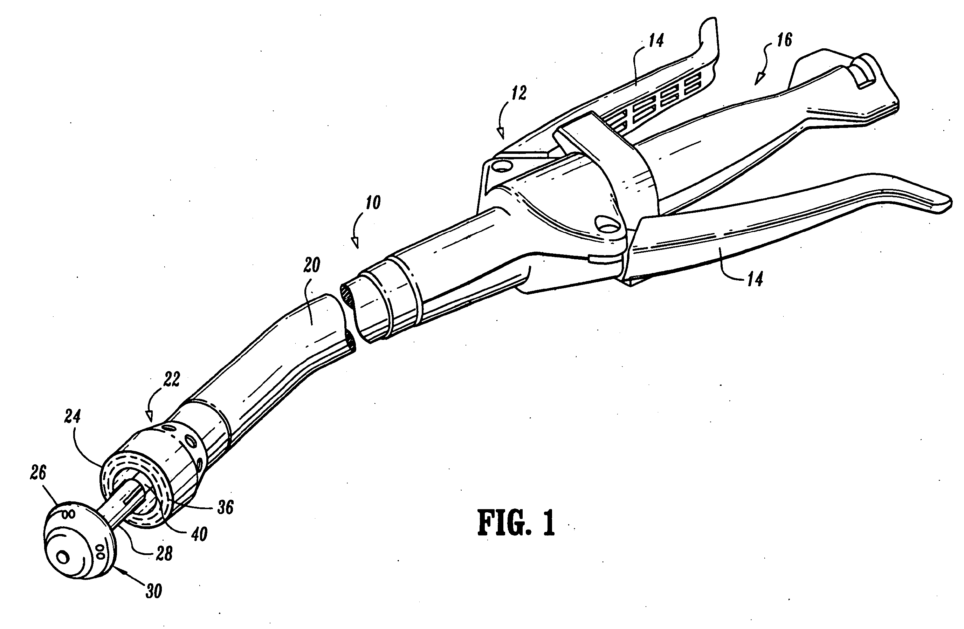

Referring initially to FIG. 1, an annular surgical stapling device, for use with the annular adhesive structures disclosed herein, is generally designated as 10. Surgical stapling device 10 includes a handle assembly 12 having at least one pivotable actuating handle member 14, and an advancing member 16. Extending from handle member 12, there is provided a tubular body portion 20 which may be constructed so as to have a curved shape along its length. Body portion 20 terminates in a staple cartridge assembly 22 which includes a pair of annular arrays of staple receiving slots 36 having a staple (not shown) disposed in each one of staple receiving slots 36. Positioned distally of staple cartridge assembly 22 there is provided an anvil assembly 30 including an anvil member 26 and an anvil rod 28 operatively associated therewith for removably connecting anvil assembly 30 to a distal end portion or connection member 40 of stapling device 10.

Staple cartridge assembly 22 may be fixedly connected to the distal end of tubular body portion 20 or may be configured to concentrically fit within the distal end of tubular body portion 20. Typically, staple cartridge assembly 22 includes a staple pusher (not shown) including a proximal portion having a generally frusto-conical shape and a distal portion defining two concentric rings of peripherally spaced fingers (not shown), each one of which is received within a respective staple receiving slot 36.

Typically, a knife (not shown), substantially in the form of an open cup with the rim thereof defining a knife edge, is disposed within staple cartridge assembly 22 and mounted to a distal surface of a staple pusher (not shown). The knife edge is disposed radially inward of the pair of annular arrays of staples. Accordingly, in use, as the staple pusher is advanced, the knife is also advanced axially outward.

Reference may be made to U.S. Patent 5,915,616 to Viola et al.. for a detailed discussion of annular stapling device 10.

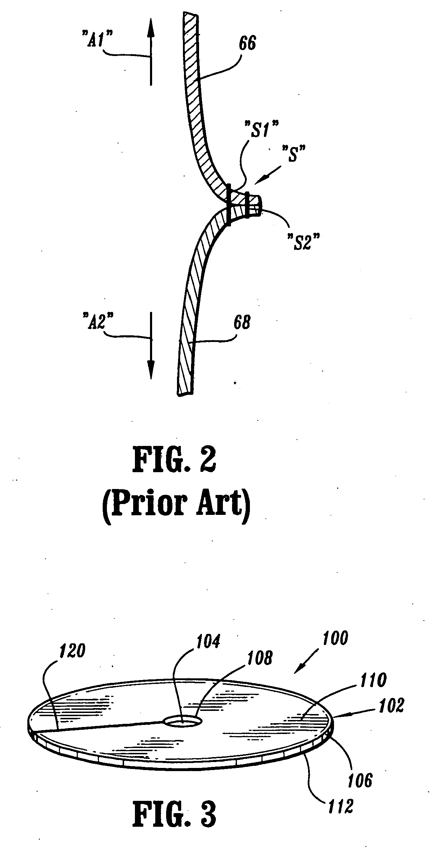

Turning now to FIG. 2, a cross-sectional schematic illustration of a pair of adjacent tissue sections 66, 68 (i.e., intestinal sections), joined to one another with annular stapling device 10 according to the method described above, is shown.

As seen in FIG. 2, when tissue sections 66 and 68 undergo a degree of tension (i.e., being pulled in opposite directions from one another), as evidenced by arrows "A1, A2", a degree of mechanical strain is placed upon staples "S". A greater degree of strain is exhibited on the radially outwardly disposed staples "S1" as compared to the radially inward disposed staples "S2". In other words, as tissues sections 66 and 68 are pulled apart, in the direction of arrows "A1, A2", a relatively high degree of strain is placed on outer staples "S1" and then on inner staples "S2". Additionally, stress concentrations are formed and/or exhibited at each outer staple "S1" of the outer row of staples.

Turning now to FIG. 3, an adhesive disk, in accordance with an embodiment of the present disclosure, is generally designated as 100. Adhesive disk 100 desirably has a shape corresponding to the arrays of staple receiving slots 36. Preferably, adhesive disk 100 includes a washer-like or gasket-like body portion 102 including a substantially centrally located aperture 104 formed therethrough. Adhesive disk 100 is defined by an outer terminal edge 106, an inner terminal edge 108 defining the size of aperture 104, an upper surface 110, and a bottom surface 112.

In one embodiment, adhesive disk 100 is sized such that when adhesive disk 100 is operatively associated with stapling device 10, as will be described in greater detail below, outer terminal edge 106 extends radially beyond staple retaining pockets 36 of staple cartridge assembly 22. Additionally, aperture 104 of structure 100 is sized to at least receive shaft 28 of anvil assembly 30 therethrough. In another embodiment, the distance between outer terminal edge 106 and inner terminal edge 108 may be substantially equal to a width of a tissue contact surface 24 (see FIG. 1) of staple cartridge assembly 22.

It is contemplated that adhesive disk 100 may be fabricated from or include a surgical grade, biocompatible, non-absorbable (i.e., permanent) material; a mesh impregnated with an adhesive. Optionally, the mesh may also be impregnated with a sealant and/or wound treatment material. For example, adhesive disk 100 may be fabricated from "TEFLON", which is a registered trademark owned by DuPont de Nemours & Co. It is further contemplated that adhesive disk 100 may be fabricated from a biocompatible polymeric foam, felt, polytetrafluoroethylene (ePTFE), gelatin, fabric or the like, or any other biocompatible material.

Non-absorbable materials used for adhesive disk 100 include, and are not limited to, those that are fabricated from such polymers as polyethylene, polypropylene, nylon, polyethylene terephthalate, polytetrafluoroethylene, polyvinylidene fluoride, and the like. Further non-absorbable materials include and are not limited to stainless steel, titanium and the like.

In one embodiment, adhesive disk 100 may be fabricated from a bio-absorbable material which is impregnated with an adhesive and, optionally also with, sealant, and/or other wound treatment material (e.g., a medicament). Accordingly, a sealant component of adhesive disk 100 can be used to retard any bleeding which may occur from the tissue, the adhesive component of adhesive disk 100 is used to secure the approximated tissue together, and the bio-absorbability of adhesive disk 100 allows for adhesive disk 100 to be absorbed into the body after a predetermined amount of time. For example, adhesive disk 100 may remain in place in the body for approximately 2-3 weeks in order for the anastomosis to sufficiently heal prior to adhesive disk 100 being absorbed into the body. In other embodiments, adhesive disk 100 has at least one portion that is absorbable and at least one portion that is not absorbable.

Bio-absorbable materials used for adhesive disk 100 include, and are not limited to, those fabricated from homopolymers, copolymers or blends obtained from one or more monomers selected from the group consisting of glycolide, glycolic acid, lactide, lactic acid, p-dioxanone, α-caprolactone and trimethylene carbonate. Other bio-absorbable materials include and are not limited to, for example, Polyglycolic Acid (PGA) and Polylactic Acid (PLA). In one embodiment, adhesive disk 100 may be fabricated from bio-absorbable felt, ePTFE, gelatin or any other bio-absorbable materials.

It is contemplated that the adhesive is a biocompatible adhesive including, but not limited to, adhesives which cure upon tissue contact, which cure upon exposure to ultraviolet (UV) light, which are two-part systems which are kept isolated from one another and cure upon coming into contact with one another, which are pressure sensitive, which are any combinations thereof, or any other known suitable adhesive. In one embodiment, it is contemplated that an adhesive having a cure time of from about 10 to 15 seconds may be used. In another embodiment, it is contemplated that an adhesive having a cure time of about 30 seconds may be used.

It is envisioned that adhesive disk 100 is impregnated with a pre-cured adhesive. The pre-cured adhesive will react with the moisture and/or heat of the body tissue to thereby activate the adhesive properties of the adhesive. It is envisioned that the pre-cured adhesive may be a hydro-gel or the like.

It is envisioned that the wound treatment material "W" includes and is not limited to one or a combination of adhesives, hemostats, sealants, coagulants, astringents, and medicaments. Other surgically biocompatible wound treatment materials "W" which may be employed in or applied by surgical instruments, including surgical staplers, include adhesives whose function is to attach or hold organs, tissues or structures; sealants to prevent fluid leakage; hemostats to halt or prevent bleeding; coagulants, astringents (e.g., sulfates of aluminum) and medicaments. Examples of adhesives which can be employed include protein derived, aldehyde-based adhesive materials, for example, the commercially available albumin/glutaraldehyde materials sold under the trade designation BioGlue^{™} by Cryolife, Inc., and cyanoacrylate-based materials sold under the trade designations Indermil^{™} and Derma Bond^{™} by Tyco Healthcare Group, LP and Ethicon Endosurgery, Inc., respectively. Examples of sealants, which can be employed, include fibrin sealants and collagen-based and synthetic polymer-based tissue sealants. Examples of commercially available sealants are synthetic polyethylene glycol-based, hydrogel materials sold under the trade designation CoSeal^{™} by Cohesion Technologies and Baxter International, Inc. Examples of hemostat materials, which can be employed, include fibrin-based, collagen-based, oxidized regenerated cellulose-based and gelatin-based topical hemostats. Examples of commercially available hemostat materials are fibrinogen-thrombin combination materials sold under the trade designations CoStasis^{™} by Tyco Healthcare Group, LP, and Tisseel^{™} sold by Baxter International, Inc.

The wound treatment material may include a cross-linking material and/or reactive agent that reacts with the annular structure, tissue or both. The resulting material acts as a seal or tissue-joining material that is non-absorbable. For example, the wound treatment material may be based on biocompatible cross-linked polymers formed from water soluble precursors having electrophilic and nucleophilic groups capable of reacting and cross-linking in situ, including those disclosed in U.S. Patent No. 6,566,406.

The wound treatment material may be disposed on adhesive disk 100 and/or impregnated into adhesive disk 100. Medicaments may include one or more medically and/or surgically useful substances such as drugs, enzymes, growth factors, peptides, proteins, dyes, diagnostic agents or hemostasis agents, monoclonal antibodies, or any other pharmaceutical used in the prevention of stenosis.

Wound treatment material "W" may include visco-elastic film forming materials, cross-linking reactive agents, and energy curable adhesives. It is envisioned that wound treatment material "W", and in particular, adhesive may be cured with the application of water and/or glycerin (e.g., 1,2,3-pranatetriol, also known as glycerol and glycerine) thereto. In this manner, the water and/or glycerin cure the adhesive and hydrate the wound.

In one embodiment, it is contemplated that adhesive disk 100 may be impregnated with a first component of a two-part adhesive and that the device deploys the second component of the two-part adhesive. For example, in a surgical stapler 10, the staples "S", which are retained in staple receiving slots 36 of staple cartridge assembly 22, may be coated with a second component (e.g., a reactant) of the two-part adhesive. In this manner, the first component of the adhesive is activated when the staples "S" penetrate and capture adhesive disk 100 during the firing sequence of surgical stapling device 10, and the two components of the adhesive contact one another.

It is further envisioned that adhesive disk 100 may be single layered including a homogeneous array of bio-absorbable or non-absorbable materials or a heterogeneous array of bio-absorbable and/or non-absorbable materials. In certain embodiments, adhesive disk 100 may be impregnated with a pressure sensitive adhesive which is activated when adjacent layers of tissue are approximated, with adhesive disk, 100 disposed therebetween.

In an alternate embodiment, it is contemplated that adhesive disk 100 may be layered, i.e., having at least two layers. In this embodiment, each layer may include a homogeneous or heterogeneous array of bio-absorbable and/or non-absorbable materials. It is envisioned that each layer may be separated from one another prior to the surgical procedure.

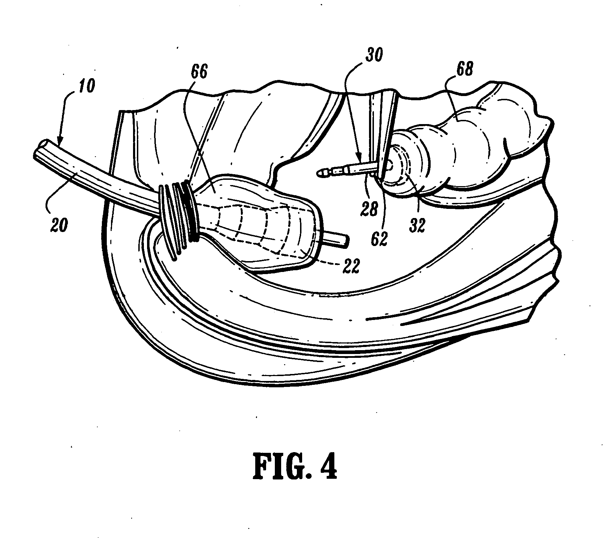

Turning now to FIGS. 4-6, there is illustrated the use of surgical stapling device 10 and adhesive disk 100 in an anastomosis procedure to effect joining of intestinal sections 66 and 68. The anastomosis procedure is typically performed using minimally invasive surgical techniques including laparoscopic means and instrumentation. At the point in the procedure shown in FIG. 4, a diseased intestinal section has been previously removed, anvil assembly 30 has been introduced to the operative site either through a surgical incision or trans-anally and positioned within intestinal section 68, and tubular body portion 20 of surgical stapling device 10 has been inserted trans-anally into intestinal section 66. Intestinal sections 66 and 68 are also shown temporarily secured about their respective components (e.g., shaft 28 of anvil assembly 30, and the distal end of tubular body portion 20) by conventional means such as a purse string suture "P".

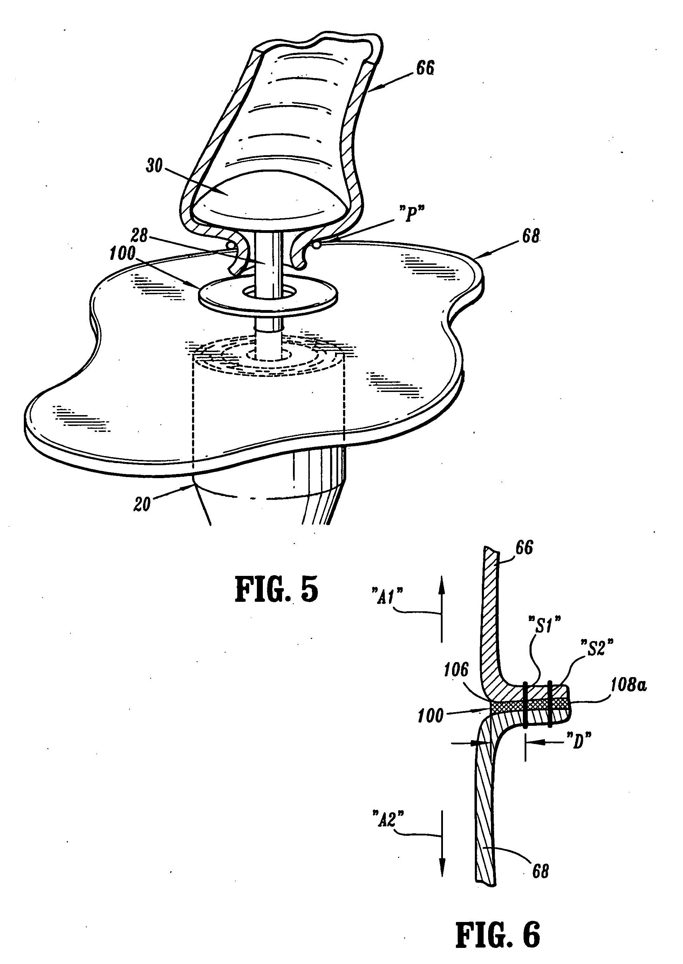

As seen in FIG. 5, adhesive disk 100 is then placed onto shaft 28 of anvil assembly 30 prior to the coupling of anvil assembly 30 to the distal end of tubular body portion 20 in order for adhesive disk 100 to be located between intestinal sections 66 and 68. In particular, shaft 28 of anvil assembly 30 is inserted through aperture 104 of adhesive disk 100. In this position, adhesive disk 100 is located adjacent intestinal section 68. Following positioning of adhesive disk 100 onto shaft 28 of anvil assembly 30, the surgeon maneuvers anvil assembly 30 until the proximal end of shaft 28 is inserted into the distal end of tubular body portion 20 of surgical stapling device 10, wherein the mounting structure (not shown) within the distal end of tubular body portion 20 engages shaft 28 to effect the mounting.

Thereafter, anvil assembly 30 and tubular body portion 20 are approximated to approximate intestinal sections 66, 68 and capture adhesive disk 100 therebetween. As seen in FIG. 6, outer terminal edge 106 of adhesive disk 100 extends radially outward, a distance "D", beyond the outer-most row of staples "S1". With adhesive disk 100 captured between intestinal sections 66, 68, surgical stapling device 10 may be fired thereby stapling intestinal sections 66, 68 to one another, securing adhesive disk 100 between intestinal section 66, 68, and cutting the portion of tissue and adhesive disk 100 disposed radially inward of the knife, to complete the anastomosis. As seen in FIG. 6, following cutting of the knife through adhesive disk 100, a new inner terminal edge 108a of adhesive disk 100 is defined.

It is envisioned that anvil assembly 30 and tubular body portion 20 are maintained in the approximated condition for a time sufficient for that portion of adhesive disk 100 located radially outward of the outer-most row of staples "S1" to adhere and/or bond with each intestinal section 66 and 68.

As seen in FIG. 6, any tension which may be experienced by intestinal sections 66 and 68, as illustrated by arrows "A1, A2" directed in opposite directions from one another, is initially absorbed by adhesive disk 100 in the location radially outward of the outer-most row of staples "S1". In this manner, the degree of strain exhibited on the outer-most row of staples "S1" is reduced as compared to when no adhesive disk 100 is present between intestinal sections 66 and 68. In other words, as intestinal sections 66 and 68 are pulled apart, in the direction of arrows "A1, A2", a relatively low degree of strain is placed on the outer-most row of staples "S1" and an even lower degree of strain is placed on the inner-most row of staples "S2". Additionally, stress concentrations at each outer staple "S1" of the outer row of staples is reduced by the inclusion of adhesive disk 100 between intestinal sections 66 and 68.

It is envisioned and understood that the greater the distance "D" that adhesive disk 100 extends beyond the outer-most row of staples "S1", the less the degree of strain which is placed on the outer-most row of staples "S1".

As seen in FIG. 3, it is contemplated that adhesive disk 100 may include a slit 120 extending between inner terminal edge 108 and outer terminal edge 106 thereby enabling adhesive disk 100 to be positioned between intestinal sections 66 and 68 following connection of anvil assembly 30 and tubular body portion 20 of surgical stapling device 10.

## Claims

1. An apparatus for forming an anastomosis between adjacent tissue sections (66, 68), the apparatus comprising:
a) an anastomosis device (10) including a tubular body portion (20) and an anvil assembly (30) having a shaft which is selectively attachable to the tubular body portion (20) wherein the tubular body portion includes a staple cartridge and at least one annular row of staples operatively disposed therein, and
b) a disk (100) having an outer terminal edge (106) and a substantially centrally located aperture (104), the disk having a mesh impregnated with an adhesive material at least at a portion of the disk, wherein said portion of the disk is the portion that extends radially outward beyond the outermost row of the at least one annular row of staples to adhesively attach the tissue sections together radially outward of the at least one annular row of staples, the disk being disposed on the shaft so that when the anvil assembly and the tubular body portion are maintained in the approximated position, the outer terminal portion of the disk is disposed between the anvil assembly and the tubular body portion and is capable of adhering adjacent tissue sections, and the disk defining a slit (120) extending between an inner terminal edge (108) and the outer terminal edge (106).

2. The apparatus according to claim 1, wherein the adhesive disk (100) is fabricated from at least one of a bioabsorbable and a non-bioabsorbable material.

3. The apparatus according to claim 1 or 2, wherein the adhesive disk (100) further comprises a material selected from a sealant, a hemostat, and a medicament.

4. The apparatus according to any one of the preceding claims, wherein the adhesive disk (100) is capable of reducing the tension exhibited on the outer-most row of the at least one annular row of staples when the adjacent tissue sections (66, 68) are pulled away from one another.

## Patentansprüche

1. Vorrichtung zum Ausbilden einer Anastomose zwischen benachbarten Gewebeabschnitten (66,68), wobei die Vorrichtung umfasst:
a) eine Anastomosevorrichtung (10), die umfasst einen röhrenförmigen Gehäuseabschnitt (20) und eine Ambossbaugruppe (30) mit einer Welle, die wahlweise an dem röhrenförmigen Gehäuseabschnitt (20) befestigbar ist, wobei der röhrenförmige Gehäuseabschnitt ein Klammermagazin und mindestens eine ringförmige Reihe Klammern umfasst, die funktionsbereit darin angeordnet sind, und
b) eine Scheibe (100) mit einer äußeren Endkante (106) und einer im Wesentlichen mittig angeordneten Öffnung (104), wobei die Scheibe wenigstens in einem Teil der Scheibe über ein mit einem Haftmittel imprägniertes Netz verfügt, wobei der Teil der Scheibe derjenige Teil ist, der sich radial nach außen über die äußerste Reihe der mindestens einen ringförmigen Reihe Klammern erstreckt, um die Gewebeabschnitte radial außerhalb der mindestens einen ringförmigen Reihe Klammern miteinander zu verbinden, wobei die Scheibe auf der Welle so angeordnet ist, dass der äußere Endteil der Scheibe, wenn die Ambossbaugruppe und der röhrenförmige Gehäuseabschnitt in der angenäherten Position beibehalten werden, zwischen der Ambossbaugruppe und dem röhrenförmigen Gehäuseabschnitt angeordnet ist und die benachbarten Gewebeabschnitte kleben kann, und wobei die Scheibe einen Schlitz (120) bildet, der sich zwischen einer inneren Endkante (108) und der äußeren Endkante (106) erstreckt.

2. Vorrichtung nach Anspruch 1, wobei die klebende Scheibe (100) aus einem bioresorbierbaren oder einem nicht-bioresorbierbaren Material hergestellt ist

3. Vorrichtung nach Anspruch 1 oder 2, wobei die klebende Scheibe (100) darüber hinaus ein Material ausgewählt aus einem Dichtungsmaterial, einer Gefäßklemme und einem Arzneimittel umfasst.

4. Vorrichtung nach einem der vorhergehenden Ansprüche, wobei die klebende Scheibe (100) die an der äußersten Reihe der mindestens einen ringförmigen Reihe Klammern beim Auseinanderziehen der benachbarten Gewebeabschnitte (66,68) entstandene Spannung reduzieren kann.

## Revendications

1. Appareil pour former une anastomose entre des sections de tissu adjacentes (66, 68), l'appareil comprenant :
a) un dispositif d'anastomose (10) comprenant une partie de corps tubulaire (20) et un ensemble d'enclume (30) ayant une tige qui peut être sélectivement fixée sur la partie de corps tubulaire (20), dans lequel la partie de corps tubulaire comprend une cartouche d'agrafes et au moins une rangée annulaire d'agrafes disposée de manière opérationnelle à l'intérieur de cette dernière, et
b) un disque (100) ayant un bord terminal externe (106) et une ouverture positionnée de manière sensiblement centrale (104), le disque ayant une maille imprégnée avec un matériau adhésif, au moins au niveau d'une partie du disque, dans lequel ladite partie du disque est la partie qui s'étend radialement vers l'extérieur au-delà de la rangée située le plus à l'extérieur de la au moins une rangée annulaire d'agrafes afin de fixer de manière adhésive les sections de tissu ensemble radialement vers l'extérieur de la au moins une rangée annulaire d'agrafes, le disque étant disposé sur la tige de sorte que lorsque l'ensemble d'enclume et la partie de corps tubulaire sont maintenus dans la position rapprochée, la partie terminale externe du disque est disposée entre l'ensemble d'enclume et la partie de corps tubulaire et peut coller les sections de tissu adjacentes, et le disque définissant une fente (120) s'étendant entre le bord terminal interne (108) et le bord terminal externe (106).

2. Appareil selon la revendication 1, dans lequel le disque adhésif (100) est fabriqué à partir d'au moins un matériau parmi un matériau bioabsorbable et un matériau non bioabsorbable.

3. Appareil selon la revendication 1 ou 2, dans lequel le disque adhésif (100) comprend en outre un matériau choisi parmi un agent d'étanchéité, un agent hémostatique et un médicament.

4. Appareil selon l'une quelconque des revendications précédentes, dans lequel le disque adhésif (100) peut réduire la tension qui apparaît sur la rangée située le plus à l'extérieur de la au moins une rangée annulaire d'agrafes lorsque les sections de tissu adjacentes (66, 68) sont écartées l'une par rapport à l'autre.
